# NEUE EUROPÄISCHE PATENTSCHRIFT

(11) **EP 1 511 438 B2**
(45) Veröffentlichungstag und Bekanntmachung des Hinweises auf die Entscheidung über den Einspruch: **09.11.2011**
(45) Hinweis auf die Patenterteilung: 09.05.2007
(21) Anmeldenummer: 03732560.2
(22) Anmeldetag: 10.06.2003
(51) Int. Cl.: A61C 1/00, A61B 18/20, A61B 18/22

(54) **MEDIZINISCHE GERÄTSCHAFTEN FÜR BEHANDLUNGEN IM DENTALEN BEREICH MITTELS EINES LASERS**
MEDICAL TOOLS FOR DENTAL TREATMENTS BY MEANS OF A LASER
APPAREIL MEDICAL POUR TRAITER LA ZONE DENTAIRE AU MOYEN D'UN LASER

(30) Priorität: 10.06.2002 DE 10225749
(43) Veröffentlichungstag der Anmeldung: 09.03.2005
(73) Patentinhaber: elexxion GmbH, 78315 Radolfzell (DE)
(72) Erfinder: Schäfer, Olaf, 78224 Singen (DE)
(74) Vertreter: Weiss, Peter
(86) Internationale Anmeldenummer: PCT/EP2003/006084
(87) Internationale Veröffentlichungsnummer: WO 2003/103529

(56) Entgegenhaltungen:
- EP-A2- 0 780 097
- WO-A-90/12548
- WO-A-99/22667
- WO-A-99/55243
- WO-A1-98/05985
- DE-A1- 19 844 719
- DE-A1- 19 855 438
- US-A- 5 346 489
- US-A- 5 401 171
- US-B1- 6 270 342
- Gebrauchsanweisung ISKRA-Twinlight Dentallaser
- Gutachten Nr. 94001/5(2) des Laser-Medezin-Zentrums Gmbh, Berlin, zur Vorlage bei der Behörde zur Erreichung einer Bauartzulassung eines medizinisch-technischen Gerätes.

## Beschreibung

Die vorliegende Erfindung betrifft eine medizinische Gerätschaft für Behandlungen im dentalen Bereich entsprechend dem Oberbegriff von Anspruch 1.

Im Bereich der Zahnmedizin gibt es derzeit fünf verschiedene Lasertypen mit insgesamt sieben verschiedenen Wellenlängen. Beispielsweise wird in der WO 93/19684 ein dentaler Laser mit einem Handstück gezeigt. Mit einem derartigen Laser ist jedoch immer nur eine einzige Behandlungsmethode möglich. Das gleiche gilt auch für eine medizinische Gerätschaft entsprechend der EP 0 523 506 A1, bei der noch zusätzlich vorgesehen ist, dass sich im Handstück Kanäle für ein Kühlmittel befinden.

Aus der DE 198 44 719 A1 ist eine Laserbehandlungsvorrichtung, insbesondere zur Durchführung einer medizinischen oder chirurgischen Behandlung mit einer Bestrahlung mittels Laserstrahlen bekannt. Diese Vorrichtung umfasst einen Festkörperlaser zur Erzeugung eines Laserstrahls, eine Anregungslichtquelle, die den Festkörperlaser anregt, ein erstes optisches System mit einem Güteschalter, das vom Festkörperlaser erzeugte Lichtwellen als einen gepulsten Laserstrahl aussendet, ein zweites optisches System, das vom Festkörperlaser erzeugte Lichtwellen als einen Dauerstrichlaser aussendet, sowie ein System zur Umschaltung des optischen Pfades, das einen optischen Pfad für die vom Festkörperlaser erzeugten Lichtschwingungen umschaltet von einem der optischen Pfade des ersten oder des zweiten optischen Systems.

In der US 6,270,342 B1 wird ein Handstück speziell für zahnmedizinische Behandlungen vorgeschlagen. In dem Handstück ist eine Funktionseinrichtung vorgegeben, die beispielsweise ein Diodenlaser, ein diodengepumpter Festkörperlaser, eine LED, eine Mikrowelleneinrichtung oder Ultraschalleinrichtung sein kann. In einem Ausführungsbeispiel 6 wird dann angegeben, dass Licht von der Lasereinrichtung in zwei Lichtsysteme aufgeteilt werden kann. Das erste Lasersystem dient dem chirurgischen Eingriff, das zweite Lasersystem desinfiziert das Gewebe, um Nebeneffekte oder Blutverlust zu reduzieren.

Aus der WO 90/12548 A1 ist ferner eine dentale Laseranordnung mit zwei Lasermodulen bekannt. Beide Lasermodule sind über einen Lichtleiter mit einem Handstück verbunden, wobei jeweils nur der eine oder der andere Laser in diesen Lichtleiter eingeschaltet werden kann.

Aus der US 5 290 274 A ist ein Lasergerät für medizinische und dentale Behandlungen bekannt. Dieses weist ein Lasersystem mit zwei Lasern unterschiedlicher Wellenlänge und dazugehörigen Lichtleitern auf, die in ein Handstück führen.

Der vorliegenden Erfindung liegt die Aufgabe zugrunde, eine medizinische Gerätschaft der o. g. Art zu entwickeln, welche dem Zahnarzt die Möglichkeit bietet, mit im wesentlichen ein und derselben Gerätschaft unterschiedliche Behandlungen im dentalen Bereich vornehmen zu können und dabei vielfältigere Behandlungsmöglichkeiten hat.

Zur Lösung dieser Aufgabe führt, die medizinische Gerätschaft nach Anspruch 1.

Dadurch, dass für das erste Lasermodul ein kurzwelliger Laser und für das zweite Lasermodul ein langwelliger Laser gewählt wird, können etwa 90 bis 95% aller gewünschten Behandlungen mit ein und demselben Gerät durchgeführt werden. Dies bietet für den Zahnarzt klare Anwendungsvorteile, so dass er bereit ist, in eine derartige Gerätschaft zu investieren.

Bei dem ersten Modul handelt es sich um ein solches für einen Diodenlaser mit einer Wellenlänge von 750 bis 1100nm. Weiter eingeschränkt liegt die Wellenlänge bevorzugt bei 810 +/- 10nm oder 980 +/- 10nm. Die Leistung liegt bei 1 bis 20W.

Bei dem zweiten, langwelligen Laser handelt es sich bevorzugt um einen Erbium: YAG-Laser in einem Wellenlängenbereich von 2500 bis 3500nm. Bevorzugt wird hier eine Wellenlänge von 2940 +/- 100nm.

Beide Lichtleiter sind separat in getrennten Handstücken vorgesehen sein. Für den Diodenlaser wird dabei Glasfaser als Lichtleiter bevorzugt, für den Erbium: YAG-Laser ein Hohlleiter.

Dem Diodenlaser soll bevorzugt noch ein optisches Element aus mindestens zwei Linsen zugeordnet sein. Desweiteren ist vorgesehen, dass sich in dem Handstück auch eine Leitung für ein Kühlmittel befindet.

Wie oben erwähnt stehen dem Zahnarzt mehrere Handstücke mit unterschiedlichen Lichtleiter und/oder Kühlmittelleitungs-Kombinationen zur Verfügung. Dies hat den Vorteil, dass der Zahnarzt nicht versehentlich falsche Funktionen in Gang setzt, da er immer das gewünschte Handstück auswählen muss und nicht versehentlich über die falsche Schaltung nicht gewünschte Funktionen auslöst.

Das Handstück ist lösbar mit einem Rückteil verbunden, welches wiederum über eine Gemeinschaftsleitung mit einem Basisgerät in Verbindung steht. In diesem Basisgerät sind die Lasermodule, die dazugehörige Leistungselsktronik und Steuerungsmodule zusammengefasst. Ferner können sich an dem Basisgerät noch ein Display und/oder Touch-Panel befinden, mit dem der Zahnarzt bestimmte Funktionen auswählen kann.

Weitere Vorteile, Merkmale und Einzelheiten der Erfindung ergeben sich aus der nachfolgenden Beschreibung bevorzugter Ausführungsbeispiele sowie anhand der Zeichnung; diese zeigt in
Figur 1 eine blockschaltbildliche Draufsicht auf einen Teil der erfindungsgemässen medizinischen Gerätschaft für Behandlungen von dentalen Bereichen mittels eines Lasers;
Figur 2 a) bis d) schematische Draufsichten auf weitere Teile der medizinischen Gerätschaft für Behandlungen im dentalen Bereichen mittels eines Lasers.

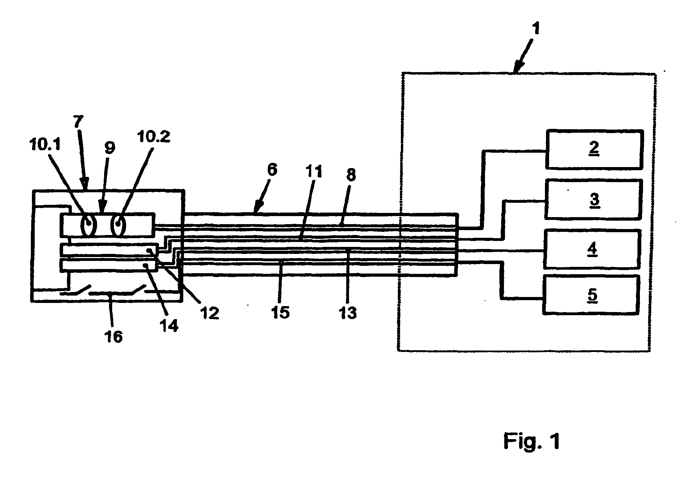

Gemäss Figur 1 befinden sich in einem Basisgerät 1 ein Lasermodul 2 eines Diodenlasers und ein Lasermodul 3 eines Erbium: YAG-Lasers. Ferner ist eine Quelle 4 für ein Kühlmittel sowie eine Steuerungselektronik 5 vorgesehen.

Das Basisgerät 1 steht über eine Gemeinschaftsleitung 6 mit einem Rückteil 7 in Verbindung. Das Dioden-Lasermodul 2 ist über eine Verbindungsleitung 8 mit einem optischen Element 9 verbunden, in das zwei Linsen 10.1 und 10.2 eingebaut sind.

Das Erbium: YAG-Lasermodul 3 steht über eine Verbindungsleitung 11 mit einem Hohlleiter 12 in Verbindung, der bevorzugt ein Edelstahlrohr aufweist, in dem das Laserlicht reflektiert wird.

Eine Verbindungsleitung 13 von der Quelle 4 für Kühlmittel mündet in ein Übertragungsstück 14. Eine Verbindungsleitung 15 von der Steuerungselektronik 5 steht mit Schaltelementen 16 für die Funktionsauswahl in Verbindung.

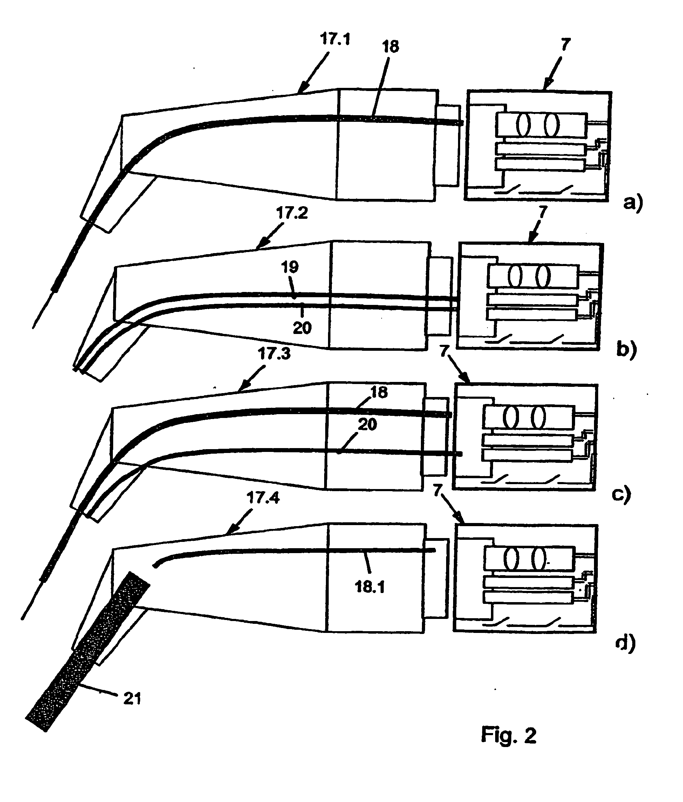

Gemäss Figur 2 können in das Rückteil 7 unterschiedliche Handstücke eingesetzt werden. Das Handstück 17.1 ist für einen Alleinbetrieb eines Diodenlasers geeignet und besitzt deshalb nur einen Lichtleiter 18. Dieser Diodenlaser soll eine hohe Leistung, typischerweise 3 bis 20 W aufweisen.

In einem Handstück 17.2 gemäss Figur 2 b) ist ein Lichtleiter 19 für den Erbium: YAG-Laser und eine Kühlmittelleitung 20 integriert.

In einem Handstück 17.3 gemäss Figur 2 c) ist dagegen der Lichtleiter 18 für den Diodenlaser mit der Kühlmittelleitung 20 kombiniert.

Bei dem Handstück 17.4 gemäss Figur 2 d) handelt es sich um einen Softlaser mit niedriger Leistung, ca. 100mW, bei dem einem Lichtleiter 18.1 ein grossflächiger Glasstab 21 zugeordnet ist, der einen Durchmesser von 5 bis 8mm haben kann. Mit ihm können insbesondere grössere dentale Bereiche einer sanften Behandlung unterzogen werden.

Die Funktionsweise der vorliegenden Erfindung ist folgende:

Das Basisgerät 1 mit den entsprechenden Lasermodulen 2 und 3, der Quelle für Kühlmittel 4 und der Steuerungselektronik 5 steht neben einem Behandlungsstuhl. Dem behandelnden Arzt steht das Rückteil 7 verbunden über die Gemeinschaftsleitung 6 mit dem Basisgerät 1 zur Verfügung. Er kann jetzt für die Behandlung eines Patienten ein ihm aus einem Bausatz zur Verfügung stehendes Handstück 17.1 bis 17.4 auswählen, je nach dem, welcher Behandlung er den Patienten unterziehen will. Über die Schaltelemente 16 am Rückteil 7 kann er dann die entsprechenden Lichtleiter 18, 18.1, 19 bzw. die Kühlmittelleitungen 20 in Funktion setzen. Selbstverständlich ist dies auch über einen entsprechenden Fussschalter möglich.

### Positionszahlenliste

| | | | | | |
|---|---|---|---|---|---|
| 1 | Basisgerät | 34 | | 67 | |
| 2 | Dioden-Lasermodul | 35 | | 68 | |
| 3 | Erbium: YAG-Lasermodul | 36 | | 69 | |
| 4 | Quelle für Kühlmittel | 37 | | 70 | |
| 5 | Steuerungselektronik | 38 | | 71 | |
| 6 | Gemeinschaftsleitung | 39 | | 72 | |
| 7 | Rückteil | 40 | | 73 | |
| 8 | Verbindungsleitung | 41 | | 74 | |
| 9 | optisches Element | 42 | | 75 | |
| 10 | Linsen | 43 | | 76 | |
| 11 | Verbindungsleitung | 44 | | 77 | |
| 12 | Hohlleiter | 45 | | 78 | |
| 13 | verbindungsleitung | 46 | | 79 | |
| 14 | übertragungsstück | 47 | | | |
| 15 | Verbindungsleitung | 48 | | | |
| 16 | Schaltelemente | 49 | | | |
| 17 | Handstück | 50 | | | |
| 18 | Lichtleiter | 51 | | | |
| 19 | Lichtleiter | 52 | | | |
| 20 | Kühlmittelleitung | 53 | | | |
| 21 | Glasstab | 54 | | | |
| 22 | | 55 | | | |
| 23 | | 56 | | | |
| 24 | | 57 | | | |
| 25 | | 58 | | | |
| 26 | | 59 | | | |
| 27 | | 60 | | | |
| 28 | | 61 | | | |
| 29 | | 62 | | | |
| 30 | | 63 | | | |
| 31 | | 64 | | | |
| 32 | | 65 | | | |
| 33 | | 66 | | | |

## Patentansprüche

1. Medizinische Gerätschaft für Behandlungen im dentalen Bereich mittels Lasers, dessen Lichtleiter (18, 19) in einem Handstück (17.1 bis 17.4) geführt ist, wobei einem ersten Lasermodul (2) ein zweites Lasermodul (3) zugeordnet ist und es sich bei dem ersten Lasermodul (2) um einen kurzwelligen Diodenlaser mit einer Wellenlänge von 750 bis 1100 nm und eine Leistung von 1 bis 10 W und bei dem zweiten Lasermodul (3) um einen langwelligen Laser, insbesondere einen Erbium: YAG-Laser, handelt und jedes Lasermodul (2, 3) eine Verbindung zu einem Lichtleiter (18, 19) und zu einer Leistungselektronik aufweist, **dadurch gekennzeichnet, dass** das erste Lasermodul (2) mit einem Lichtleiter (18) verbunden ist, während das zweite Lasermodul (3) mit einem separaten Lichtleiter (19) verbunden ist, wobei jedem Lasermodul (2, 3) ein eigener Lichtleiter (18, 19) separat in getrennten Handstücken (17.1 bis 17.4) zugeordnet ist, und dass das Handstück (17.1 bis 17.4) lösbar mit einem Rückteil (7) verbunden ist, wobei das Rückteil (7) mehrere Handstücke (17.1 bis 17.4) mit unterschiedlichen Lichtleitern und/oder Kühlmittelleitungs-Kombinationen zugeordnet sind und dieses eine Rückteil (7) über eine Gemeinschaftsleitung (6), welche Verbindungsleitungen (8, 11) zwischen den beiden Lasermodulen (2, 3) und dem Rückteil (7) umfasst, mit einem Basisgerät (1) in Verbindung steht, in dem sich die Lasermodule (2, 3) befinden.

2. Gerätschaft nach Anspruch 1, **dadurch gekennzeichnet, dass** der Laser des zweiten Lasermoduls (3) eine Wellenlänge von 2500 bis 3500nm aufweist.

3. Gerätschaft nach Anspruch 1 oder 2, **dadurch gekennzeichnet, dass** dem ersten Lasermodul (2) Glasfaser als Lichtleiter zugeordnet ist.

4. Gerätschaft nach wenigstens einem der Ansprüche 1 bis 3, **dadurch gekennzeichnet, dass** dem zweiten Lasermodul (3) ein Hohlleiter (12) als Lichtleiter zugeordnet ist.

5. Gerätschaft nach Anspruch 4, **dadurch gekennzeichnet, dass** in den Lichtleiter bzw. in eine Verbindungsleitung (8) des ersten Lasermoduls (2) ein optisches Element (9) aus mindestens zwei Linsen (10.1, 10.2) eingeschaltet ist.

6. Gerätschaft nach wenigstens einem der Ansprüche 1 bis 5, **dadurch gekennzeichnet, dass** in dem Handstück (17.2, 17.3) eine Leitung (20) für ein Kühlmittel vorgesehen ist.

7. Gerätschaft nach wenigstens einem der Ansprüche 1 bis 6, **dadurch gekennzeichnet, dass** im Rückteil (7) das optische Element (9) mit den zwei Linsen (10.1, 10.2), der Hohlleiter (12), eine Leitung (14) für das Kühlmittel und Schaltelemente (16) für die Laser und das Kühlmittel vorgesehen sind.

8. Gerätschaft nach wenigstens einem der Ansprüche 1 bis 7, **dadurch gekennzeichnet, dass** beide Lasermodule (2, 3) zusammen mit der dazu gehörigen Leistungselektronik und einem Steuerungsmodul (5) in einem Gehäuse (1) angeordnet sind.

9. Gerätschaft nach Anspruch 8, **dadurch gekennzeichnet, dass** an dem Gehäuse (1) auch ein Display und/oder ein Touch-Panel vorgesehen ist.

## Claims

1. Medical instrument for dental treatments by means of laser, whereof the light guide (18, 19) are guided in a handpiece (17.1 to 17.4), wherein associated with a first laser module (2) is a second laser module (3) and the first laser module (2) is a short-wave diode laser having a wavelength of 750 to 1100 nm and a power range of 1 to 10 W and the second laser module (3) is a long-wave laser, in particular an erbium:YAG laser, and each laser module (2, 3) has a connection to a light guide (18, 19) and to power electronics, **characterized in that** the first laser module (2) is connected to a light guide (18), while the second laser module (3) is connected to a separate light guide (19), wherein associated with each laser module (2, 3) is an individual light guide (18, 19), kept separate in separate handpieces (17.1 to 17.4), and **in that** the handpiece (17.1 to 17.4) is connected detachably to a rear part (7), wherein a plurality of handpieces (17.1 to 17.4) having different light guides and/or cooling medium line combinations are associated with the rear part (7), and this one rear part (7) is connected by way of a shared line (6) including connection lines (8, 11) between the two laser modules (2, 3) and the rear part (7) to a base unit (1) in which the laser modules (2, 3) are situated.

2. Instrument according to claim 1, **characterized in that** the laser of the second laser module (3) has a wavelength of 2500 to 3500 nm.

3. Instrument according to claim 1 or 2, **characterized in that** glass fibre is associated as a light guide with the first laser module (2).

4. Instrument according to at least one of claims 1 to 3, **characterized in that** a waveguide (12) is associated as a light guide with the second laser module (3).

5. Instrument according to claim 4, **characterized in that** an optical element (9) comprising at least two lenses (10.1, 10.2) is inserted into the light guide and/or into a connection line (8) of the first laser module (2).

6. Instrument according to at least one of claims 1 to 5, **characterized in that** a line (20) for a cooling medium is provided in the handpiece (17.2, 17.3).

7. Instrument according to one of claims 1 to 6, **characterized in that** provided in the rear part (7) are the optical element (9) with the two lenses (10.1, 10.2), the waveguide (12), a line (14) for the cooling medium, and operating elements (16) for the lasers and the cooling medium.

8. Instrument according to at least one of claims 1 to 7, **characterized in that** both laser modules (2, 3) together with their associated power electronics and a control module (5) are disposed in a housing (1).

9. Instrument according to claim 8, **characterized in that** on the housing (1) a display and/or a touch panel are also provided.

## Revendications

1. Appareil médical pour le traitement de la zone dentaire au moyen d'un laser dont le conducteur de lumière (18, 19) est guidé dans une pièce à main unique (17.1 à 17.4), à un premier module de laser (2) étant associé un deuxième module de laser (3) et où il s'agit, pour le premier module de laser (2), d'un laser à diode à ondes courtes d'une longueur d'onde de 750 à 1100 nm et d'une puissance de 1 à 10 W et, pour le deuxième module de laser (3), d'un laser à ondes longues, en particulier d'un laser à erbium : YAG, et chaque module de laser (2, 3) présentant une connexion avec un conducteur de lumière (18, 19) et avec une électronique de puissance, **caractérisé par le fait que** le premier module de laser {2) est relié à un conducteur de lumière (18), tandis que le deuxième module de laser (3) est relié à un conducteur de lumière séparé (19), à chaque module de laser (2, 3) étant associé séparément un conducteur de lumière propre (18, 19), dans des pièces à main séparées (17.1 à 17.4), et la pièces à main (17.1 à 17.4) étant assemblée de manière amovible à une pièce de dos (7), à la pièce de dos (7) étant associées plusieurs pièces à main (17.1 à 17.4) à conducteurs de lumière et/ou combinaisons de conduits de réfrigérant différents et cette une pièce de dos (7) étant en communication, par l'intermédiaire d'un conduit commun (6) comprenant des lignes de connexion (8, 11) entre les deux modules de laser (2, 3) et la pièce de dos (7), avec un appareil de base (1) dans lequel se trouvent les modules de laser (2, 3).

2. Appareil selon la revendication 1, **caractérisé par le fait que** le laser du deuxième module de laser (3) présente une longueur d'onde de 2500 à 3500 nm.

3. Appareil selon la revendication 1 ou 2, **caractérisé par le fait qu'**au premier module de laser (2) est associée une fibre de verre comme conducteur de lumière.

4. Appareil selon l'une des revendications 1 à 3, **caractérisé par le fait qu'**au deuxième module de laser (3) est associé un conducteur creux (12) comme conducteur de lumière.

5. Appareil selon la revendication 4, **caractérisé par le fait que** dans le conducteur de lumière ou dans une ligne de connexion (8) du premier module de laser (2) est intercalé un élément optique (9) composé d'au moins deux lentilles (10.1, 10.2).

6. Appareil selon au moins l'une des revendications 1 à 5, **caractérisé par le fait que** dans la pièce à main (17.2, 17.3) est prévue un conduit (20) pour un réfrigérant.

7. Appareil selon au moins l'une des revendications 1 à 6, **caractérisé par le fait que** dans la pièce de dos (7) sont prévus l'élément optique (9) avec les deux lentilles (10.1, 10.2), le conducteur creux (12), un conduit (14) pour le réfrigérant et des éléments de commutation (16) du laser et du réfrigérant.

8. Appareil selon l'une des revendications 1 à 7, **caractérisé par le fait que** les deux modules de laser (2, 3) sont disposés, ensemble avec l'électronique de puissance y relative et un module de commande (5), dans un boîtier (1).

9. Appareil selon la revendication 8, **caractérisé par le fait qu'**au boîtier (1) est également prévu un écran d'affichage et/ou un panneau tactile.
